Europäisches Patentamt

European Patent Office

Office européen des brevets

(19)

(11) Numéro de publication: **0 179 694**
**A2**

(12)

# DEMANDE DE BREVET EUROPEEN

(21) Numéro de dépôt: 85401866.0

(22) Date de dépôt: 25.09.85

(51) Int. Cl.⁴: **A 61 K 31/325**

(30) Priorité: 26.10.84 FR 8416393

(43) Date de publication de la demande:
30.04.86 Bulletin 86/18

(84) Etats contractants désignés:
AT BE CH DE FR GB IT LI NL SE

(71) Demandeur: INSTITUT MERIEUX Société anonyme dite:
17, rue Bourgelat
F-69002 LYON(FR)

(72) Inventeur: Bouzinac de la Bastide, René Marie L.G.
43 allée des Tilleuls
F-69340 Francheville(FR)

(72) Inventeur: Charbonnier, Claude Jean
8 rue de Charavey
F-69290 St Genis Les Ollieres(FR)

(72) Inventeur: Musset, Mircea
21 avenue du Général Leclerc
F-69160 Tassin La Demi Lune(FR)

(74) Mandataire: Bernasconi, Jean et al,
CABINET LEMOINE ET BERNASCONI 13, BLd. des
Batignolles
F-75008 Paris(FR)

(54) Nouveau médicament modulateur de l'immunité et son procédé de préparation.

(57) Nouveau médicament modulateur de l'immunité et son
procédé de préparation.
Le médicament comprend une préparation de diéthyldithiocarbamate de sodium purifié par dissolution dans de
l'eau.

EP 0 179 694 A2

Croydon Printing Company Ltd

Nouveau médicament modulateur de l'immunité et son procédé de préparation.

La présente invention a trait à un nouveau médicament modulateur et notamment stimulant de l'immunité ainsi qu'à un procédé de préparation de ce médicament.

On a déjà décrit, dans le brevet français 2.269.960, à titre de nouveau médicament immuno-stimulant, des thiols minéraux, parmi lesquels le diéthyldithiocarbamate de sodium (DTC). Un certain·nombre de travaux, sous l'impulsion de Gérard Renoux, ont confirmé l'intérêt du DTC à titre de médicament immuno-modulateur, dont on a étudié un certain nombre de propriétés pharmacologiques et suggéré un assez large éventail d'applications thérapeutiques. Voir :

- Gérard Renoux et al. Immunopharmacology of DTC in mice and men, Immunomodulatory Drugs and Modifiers of the Biological Response, Elsevier Biomedical Press pp 113 - 132, 1982.

- Gérard Renoux et al. Sodium diethyldithiocarbamate (Imuthiol) and cancer, Biological Response Modifiers in Human Oncology and Immunology, Ed. by Thomas Klein, Steven Specter, Herman Friedman and Andor Szentivanyl, Plenum Publishing Corporation, pp 223 - 239, 1983.

- Gérard Renoux. Recent Advances in Immunopharmacology and Immunotherapy, Recent Advances in Tumor Immunology : Oncogenes to Tumor Antigens, Anacapri, May 13-15, 1984.

Ces différents travaux ont montré l'intérêt du DTC dans le cas de maladies ou complications infectieuses, de maladies auto-immunes, dans les maladies néoplasiques et, d'une façon générale, dans les différentes situations liées à, ou provoquant, une altération de la réponse immunitaire normale.

En fait, le diéthyldithiocarbamate qui était disponible pour ces travaux était loin d'être un produit purifié et comportait une teneur importante en impuretés diverses et notamment en un contaminant toxique de couleur rougeâtre.

On a donc essayé de purifier le DTC disponible et de nouveaux travaux ont été effectués à l'aide d'une préparation purifiée à l'aide de solvants organiques. L'utilisation du DTC ainsi purifié a permis de montrer une diminution sensible de la toxicité, comme le montre - Charles Frederick Corke. T Cell subsets, functiuning B Cells and the influence of Diethyldithiocarbamate on T Cell subsets and clinical parameters in Rheumatoïd Arthritis, projet de thèse à l'Université de Londres, Department of Rheumatology St Bartholomew's Hospital, octobre 1983, non soutenue et non publiée.

Il s'est cependant avéré que les préparations de DTC ainsi purifiées comportent encore un grand nombre d'impuretés dont on pouvait penser raisonnablement que la toxicité n'était pas négligeable pour le traitement de patients plus sensibles aux médicaments, notamment les enfants et nourrissons.

Des essais de toxicité sur souris, rats et chiens effectués avec une préparation davantage purifiée et lyophilisée de DTC ont également montré une réduction de la toxicité chez ces animaux - Voir Gérard Renoux, Immunopharmacologie et Pharmacologie du Diéthyldithiocarbamate (DTC), J. Pharmacol. (Paris), 1982, 13, suppl.1 95-134.

La présente invention se propose donc de fournir un nouveau médicament à base de DTC qui soit susceptible d'être utilisé, aux doses efficaces, chez tous les types de patients y compris les enfants, les nourrissons et les personnes agées.

Un autre objectif de l'invention est de fournir un tel médicament qui, tout en ayant un large spectre d'activité, soit particulièrement utile pour la prévention et le traitement d'affections liées à des dérèglements immunitaires chez l'enfant ou le nourrisson, et notamment la mucoviscidose, les infections respiratoires récidivantes, la polyarthrite juvénile et la papillomatose laryngée de l'enfant.

Un autre objectif de l'invention est de fournir un tel médicament qui soit particulièrement actif dans le cas de maladies auto-immunes des adultes et notamment la polyarthrite rhumatoïde, le lupus érythémateux disséminé ainsi que la bronchite chronique, la bird shot rétinopathie et la maladie de Behcet, ainsi que la prévention et le traitement des dérèglements immunologiques chez les personnes agées.

Un autre objectif de l'invention est de fournir un médicament utilisable dans la prévention et le traitement du syndrome immuno-déficitaire acquis (SIDA) et des manifestations associées (ARC).

Un autre objectif de l'invention est de fournir un procédé de purification des diéthyldithiocarbamates, notamment de sodium, permettant la préparation des médicaments selon l'invention.

La présente invention a pour objet un nouveau médicament comprenant une préparation purifiée de diéthyldithiocarbamate (DTC) caractérisé en ce que le DTC a été purifié par dissolution dans un milieu aqueux.

De préférence, le médicament selon l'invention est caractérisé en ce que la purification par dissolution en milieux aqueux a été effectuée conformément au procédé défini ci-après.

Le médicament selon l'invention peut être administré de préférence par voie orale ou par voie intraveineuse.

Pour l'administration par voie orale, le médicament est de préférence conditionné sous forme de gélules gastroprotégées. On constate de façon surprenante que ces gélules restent stables dans leur présentation et dans leur coloration aux températures habituelles de conservation alors que les gélules qui avaient été préparées avec des préparations de diéthyldithiocarbamate de sodium antérieurement connues voyaient leur couleur et leur aspect se modifier et se dégrader avec le temps.

Pour l'administration par voie intraveineuse, la préparation selon l'invention est de préférence lyophilisée et prévue pour être dissoute dans un solvant spécial.

Les dosages par voie orale sont de préférence de l'ordre de 125 mg pour l'adulte et 50 mg pour l'enfant. Les dosages en flacon lyophilisé sont de préférence de l'ordre de 125 mg et 500 mg.

L'effet sur l'organisme récepteur n'est pas dose – dépendant. Cependant les doses préférées de traitement sont, par voie orale, de l'ordre de 10 mg par kg par semaine pour l'enfant et l'adulte et par voie intraveineuse de l'ordre de 5 mg par kg par semaine. De préférence, la dose hebdomadaire est administrée en une seule fois.

Le médicament selon l'invention peut être utilisé pour le rétablissement de l'activité immunitaire dans toutes sortes d'affections ou de complications en chirurgie, cure d'infections au long cours ou récurrentes et notamment de tuberculoses antibio-résistantes, complications septiques en cas de chocs, traumatismes, brûlures ; stimulations de l'immunité dans les cancers, polyarthrite rhumatoïde et lupus érythémateux disséminé, SIDA, et, plus particulièrement chez l'enfant, mucoviscidose, infections respiratoires récidivantes, polyarthrite juvénile, SIDA.

L'invention a également pour objet l'application du DTC ainsi purifié à la préparation d'un médicament utilisable dans le traitement du SIDA, y compris des manifestations associées, notamment ARC.

L'invention a également pour objet un procédé de préparation de ce médicament caractérisé en ce que l'on traite une préparation commerciale de DTC par les étapes consistant à dissoudre le DTC dans une solution aqueuse, à effectuer, de préférence après une filtration clarifiante, une filtration sur membrane stérilisante, puis à récupérer le DTC, de préférence par lyophilisation à une température inférieure à 40°C, les opérations en phase aqueuse s'effectuant pendant une durée totale de préférence inférieure à 24 h.

On a pu constater, grâce à l'invention, que l'on pouvait ainsi obtenir un degré de pureté très élevé du DTC alors qu'il s'agit d'une substance qui s'hydrolyse et se dégrade spontanément en milieu aqueux, ce qui avait dissuadé, jusqu'à présent, toute tentative de purification par voie hydrique.

De préférence, on effectue la dissolution de la préparation commerciale impure de DTC dans une quantité suffisante d'eau sous agitation pendant par exemple environ un quart d'heure, à la suite de quoi on effectue une première filtration clarifiante sur membrane de porosité 150 à 250 microns, de préférence en verre fritté, suivie d'une deuxième filtration sur membrane stérilisante 0,2 micron. On effectue ensuite une lyophilisation à température inférieure à 40°C. Pour la préparation à administrer par voie orale, on réhydrate ensuite la substance et on effectue ensuite une granulation humide de la substance au degré de granulométrie recherché. Le médicament peut alors être conditionné pour l'administration par voie orale.

Pour la préparation à administrer par voie intra-veineuse, la lyophilisation est effectuée directement dans les flacons prévus pour l'application du traitement.

D'autres avantages et caractéristiques de l'invention apparaîtront à la lecture de la description suivante, faite à titre d'exemple non limitatif.

Exemple 1 - Purification de la préparation commerciale de diéthyldithiocarbamate de sodium

La substance de départ est une préparation de diéthyldithiocarbamate de sodium en provenance de la société CLEVENOT, Darmstadt, République Fédérale Allemande. Cette préparation présente les caractéristiques physiques suivantes :

Couleur : Blanchâtre, tirant sur le jaune ou le rose.

Degré d'hydratation : 23 à 25 %.

On introduit 13 kg de cette préparation dans un volume de 50 litres d'eau pure dépyrogénée, en l'absence de toute substance métallique tant au niveau du récipient qu'au niveau de l'agitateur. La dissolution est complète, à température ambiante, après environ un quart d'heure.

On effectue ensuite une filtration clarifiante sur membrane en verre fritté de porosité 200 microns environ, toujours à température ambiante, ceci pendant une durée de 30 minutes environ.

La solution clarifiée est ensuite soumise à une filtration sur membrane stérilisante 0,2 micron.

Cette filtration s'effectue environ en 30 à 60 minutes.

On effectue ensuite une lyophilisation sous vide avec une température finale n'excédant pas 40°C. Cette lyophilisation dure environ 48 heures.

On obtient ainsi une substance lyophilisée d'un poids de 10 kg.

On réhydrate cette masse et on effectue une granulation humide de cette masse pour obtenir un degré de granulométrie de l'ordre de 800 microns.

Exemple 2 – Conditionnement

La substance est ensuite conditionnée sous forme de gélules de 50 et 125 mg.

Pour les préparations par voie intraveineuse, on effectue simplement l'étape précitée de lyophilisation directement dans les flacons prévus pour l'application.

On prépare, pour la dissolution, un liquide de dissolution dont la composition est la suivante :

| | | |
|---|---|---|
| Phosphate monosodique | $NaH_2PO_4 2H_2O$ | 1,00 g |
| Phosphate disodique | $Na_2HPO_4, 12H_2O$ | 3,70 g |
| Chlorure de sodium | $NaCl$ | 8,00 g |
| Edétate de sodium | $C_{10}H_{14}N_2Na_2O_8, 2H_2O$ | 0,5 g |
| Eau p.p.i. | QSP 1000ml | |

NaOH QS pH ≅ 7,0

Exemple 3 – Traitement de la mucoviscidose (résultats après 6 mois d'utilisation – Posologie 10 mg/kg (semaine)

Sujet n° 1. Date de naissance (DN)

17/7/68, surinfections pulmonaires fréquentes au cours de l'hiver, à staphylocoque. Cette forme évolue pour l'instant sans problème. Le DTC a débuté en avril 83. L'hiver 83-84 s'est passé sans surinfection. En juin 84, surinfection à staphylocoque et épisodes de dyspnée expiratoire améliorée par l'Euphilline. En un an, le poids est passé de 50 kg à 53 kg 800.

Sujet n° 2. DN 31/8/73 – un frère est mort à 13 ans de mucoviscidose. Il s'agit d'une forme pulmonaire majeure avec surinfection pyocyanique en permanence. DTC débuté en juin 83. Est actuellement stabilisé sur le plan de la fonction pulmonaire mais le taux de pyocyanique dans les crachats n'a pas été modifié. Est passé de 28 kg 500 à 34 kg.

Sujet n° 3. – DN 15/7/71 – forme limitée à des surinfections pulmonaires hivernales. Début du DTC en octobre 83. Hiver sans surinfection. Est passé, en un an, de 32 kg 800 à 37 kg et de 1,42 m à 1,48 m.

Sujet n° 4. – DN 19/8/78 – pathologie pulmonaire discrète pendant l'hiver. Début du DTC en septembre 83. Pas de surinfection pulmonaire cet hiver. Poids 16 kg.

0179694

Sujet n° 5. - DN 25/2/78 - forme bénigne pour l'instant : rhinopharyngites récidivantes, bon développement statural. DTC débuté en octobre 83.

Sujet n° 6 - DN 1960 - Mucoviscidose découverte à l'âge de un an, bien tolérée jusqu'en 1978. Depuis, aggravation progressive de l'insuffisance pulmonaire avec surinfection massive à staphylocoque. DTC débuté en décembre 83. Pas de modification significative de la flore. A signaler une amélioration du poids de 47 kg à 50 kg.

Sujet n° 7 - DN 13/10/73 - infections pulmonaires hivernales - hépatomégalie sans cirrhose biologique. Depuis la mise sous DTC, aucun épisode infectieux pulmonaire ou ORL. Excellent développement staturopondéral.

Sujet n° 8 - DN 24/9/70 - forme d'évolution sévère - surinfection permanente à pyocyanique. Echec de tous les antibiotiques. DTC débuté en janvier 84. Etat stationnaire depuis. Poids 28 kg.

Sujet n° 9 - Maladie évoluant depuis plus de 15 ans. Surinfection pulmonaire à pyocyanique améliorée par la Péfloxacine. DTC débuté en décembre 83. Survenue de deux poussées depuis, ayant nécessité des perfusions de Cefsulodine.

Ces exemples montrent que le médicament selon l'invention permet une bonne maîtrise des complications et améliore fortement la croissance staturo-pondérale.

Exemple 4 - <u>Traitement de l'ARC (complexe relatif au SIDA)</u>

Un essai pilote sans contrôle a été réalisé chez des patients mâles complètement informés sous administration hebdomadaire de 5-10 mg/kg de poids corporel pendant 3 à 6 mois avec les résultats suivants.

$T_4$ + PBL (lymphocytes du sang périphérique)
chez des patients ARC.

| Patients | avant | après | durée du traitement |
|---|---|---|---|
| 1 | 10 % (102) | 42 % (672) | 5 mois |
| 2 | 24 % (494) | 21 % (504) | 6 mois |
| 3 | 43 % (2400) | 50 % (1275) | 6 mois |
| 4 | 14 % (157) | 22 % (330) | 3 mois |
| 5 | 26 % (468) | 33 % (267) | 4 mois |
| 6 | 34 % (697) | 33 % (429) | 6 mois |

$T_8$ + PBL chez des patients ARC

| Patients | avant | après | durée du traitement |
|---|---|---|---|
| 1 | 17 % (173) | 35 % (560) | 5 mois |
| 2 | 21 % (432) | 30 % (720) | 6 mois |
| 3 | 18 % (1008) | 23 % (586) | 6 mois |
| 4 | 8 % (90) | 10 % (150) | 3 mois |
| 5 | 33 % (594) | 22 % (178) | 4 mois |
| 6 | 32 % (656) | 17 % (221) | 6 mois |

0179694

### E-ROSETTES chez des patients ARC

| Patients | avant | après | durée du traitement |
|---|---|---|---|
| 1 | 33 % (337) | 70 % (1120) | 5 mois |
| 3 | 39 % (2184) | 64 % (1632) | 6 mois |
| 4 | 34 % (382) | 73 % (1095) | 3 mois |
| 5 | 55 % (900) | 66 % (534) | 4 mois |

### DCH chez des patients ARC
### DCH score (hypersensibilité retardée)

| Patients | avant | après | durée du traitement |
|---|---|---|---|
| 1 | 0 | 8,5 | 5 mois |
| 2 | 7,5 | 17,5 | 6 mois |
| 3 | 0 | 7 | 6 mois |
| 4 | 2,5 | 2,5 | 3 mois |
| 6 | 0 | 23 | 6 mois |

Ces résultats montrent l'effet positif du médicament selon l'invention, montré par la surveillance des paramètres immunologiques et l'amélioration clinique relevée chez tous les patients.

En outre on pense que le médicament selon l'invention est utile dans le traitement des désordres immunitaires (y compris les déficiences de lymphocytes T).

## REVENDICATIONS

1) Médicament comprenant une préparation purifiée de diéthyldithiocarbamate (DTC), caractérisé en ce que le DTC a été purifié par dissolution dans un milieu aqueux.

2) Médicament selon la revendication 1, caractérisé en ce qu'il comporte du diéthyldithiocarbamate de sodium.

3) Médicament selon l'une des revendications 1 et 2, caractérisé en ce qu'il est conditionné sous forme de gélules gastroprotégées.

4) Médicament selon la revendication 3, caractérisé en ce que les gélules sont dosées à 125 ou 50 mg.

5) Médicament selon l'une des revendications 1 et 2, caractérisé en ce qu'il est conditionné sous forme de flacons lyophilisés.

6) Procédé de préparation du médicament selon l'une quelconque des revendications 1 à 5, caarctérisé en ce que l'on purifie le DTC par dissolution dans un milieu aqueux.

7) Procédé selon la revendication 6, caractérisé en ce que l'on traite une préparation commerciale de DTC par les étapes consistant à dissoudre le DTC dans une solution aqueuse, à effectuer une filtration sur membrane stérilisante puis à récupérer le DTC.

8) Procédé selon la revendication 7, caractérisé en ce que l'on effectue une filtration clarifiante avant la filtration stérilisante.

9) Procédé selon la revendication 8, caractérisé en ce que l'on dissout la préparation commerciale de DTC dans une quantité suffisante d'eau sous agitation, on effectue une filtration clarifiante sur membrane 150 à 250 microns, suivie d'une filtration stérilisante sur membrane 0,2 micron, puis une lyophilisation à une température inférieure à 40°C.

10) Procédé selon l'une quelconque des revendications 6 à 9, caractérisé en ce que la solution aqueuse utilisée est de l'eau.

11) Médicament selon l'une des revendications 1 à 5 pour l'utilisation dans la prévention ou le traitement des affections ou syndromes suivants : mucoviscidose, infections respiratoires récidivantes, polyarthrite juvénile, papillome laryngé de l'enfant, polyarthrite rhumatoïde, lupus érythémateux disséminé, SIDA (syndrome d'immunodéficience acquise) et ARC, infe: :ions au long cours ou infections récidivantes, complications infectie :ces, bronchite chronique, bird shot rétinopathie, maladie de Behcet, et d'une façon générale les désordres immunitaires notamment de l'enfant ou nourrisson et des personnes agées, y compris les déficiences de lymphocytes T, et pour la stimulation de l'immunité.

12) Application d'une préparation purifiée de diéthyldithiocarbamate (DTC), dans laquelle le DTC a été purifié par dissolution dans un milieu aqueux, à la préparation d'un médicament utilisable dans la prévention et le traitement du SIDA, y compris des manifestations associées ARC.